# EUROPEAN PATENT APPLICATION

(11) **EP 1 882 485 A2**
(43) Date of publication of application: **30.01.2008**
(21) Application number: 06126049.3
(22) Date of filing: 13.12.2006
(51) Int. Cl.: A61M 5/14

(54) **Mobile drip stand and method for storing the same**

(30) Priority: 27.12.2005 NL 1030782
(71) Applicant: Endomed B.V., 6942 GS Didam (NL)
(72) Inventor: Hessels, Martijn Jacobus, 1013 TH, Amsterdam (NL)
(74) Representative: Vernout, Robert

(57) **Abstract**

A mobile drip stand comprising a substantially horizontally extending frame, which frame comprises a front half with downwardly extending front wheels and a rear half with downwardly extending rear wheels, wherein an opening which is wider than the front side of the front half of the frame with the front wheels is present between the rear wheels, wherein a post extends substantially vertically from the frame, which post is provided with means for suspending a drip bag therefrom, and wherein the post extends from the rear half of the frame, and in that the height of the upper side of the frame at the front side thereof is lower than the height of the bottom side of the frame under the post, such that the front side of the frame of the drip stand can be moved between the rear wheels and under the post of a second, identical drip stand on a flat floor without lifting one of the drip stands.

## Description

The invention relates to a mobile drip stand comprising a substantially horizontally extending frame, which frame comprises a front half with downwardly extending front wheels and a rear half with downwardly extending rear wheels, whilst an opening which is wider than the front side of the front half of the frame with the front wheels is present between the rear wheels and a post extends substantially vertically from the frame, which post is provided with means for suspending a drip bag therefrom. Such a drip stand is described in WO 2004/101034.

Mobile drip stands are commonly known, they usually comprise a frame similar to that of a desk chair, i.e. a frame consisting of five legs which extend in substantially horizontal, radial direction from a centre and which are each provided with a downwardly extending castor at their ends. A post provided with suspension means for one or more drip bags extends in vertical direction from the centre of the frame. Furthermore, a pump may be attached to the post. A hospital generally has a stock of drip stands which, insofar as they are not being used, are stored in a storeroom, for example. The drip stand essentially has two functions, viz. to support drip bags and pumps , and to provide stability and mobility for the patient, both for walking patients and for wheelchair patients. The drip stand of WO 2004/101034 aims at providing an improved stability for users, inter alia by using a different frame configuration.

The object of the invention is to provide an improved drip stand in the light of one or more of the above functions and situations.

In order to accomplish that object, the post preferably extends from the rear half of the frame, and the height of the upper side of the frame at the front side thereof is lower than the height of the bottom side of the frame under the post. Because of these aspects, it is possible to move the front side of the frame of the drip stand between the rear wheels and under the post of a second, identical drip stand on a flat floor without lifting one of the drip stands. As a result, the drip stands take up significantly less space than the existing drip stands. Furthermore, because of this aspect the front side of the drip stand can be moved relatively far under the rear side of a wheelchair in most cases, so that the drip stand can be easily taken along when the patient is being wheeled through the hospital, without hindering the person who pushes the wheelchair.

In the preferred embodiment, the front side of the frame extends between two spaced-apart front wheels.

The frame is preferably made of tube members. Preferably, the frame comprises two more or less parallel, substantially horizontally extending tube members, which are interconnected and which are held in spaced-apart relationship by a bridge member, in such a manner that the frame is substantially H-shaped, seen from above, with the wheels extending downwards near the ends of the two tube members. The post preferably extends from said bridge member. In this way a space is formed in the frame on two sides of the post for a person, i.e. the patient or a nursing attendant, to move his or her feet in while walking, without stumbling over projecting legs, or which can be used for positioning the frame round another object.

The tube members preferably slope downwards between the post and the front side of the frame, preferably in directions towards each other, so as to create the aforesaid compact storage possibility. Furthermore it is possible to lift the front tube members with the front wheels over the bridge member and position them between the front tube members of a second, identical drip stand upon storage, thereby interlocking the drip stands, which may be advantageous when transporting several drip stands.

The post is preferably slightly upwardly inclined towards the rear side of the frame along at least part of its length. As a result, additional space is created when walking, so that the person moving the drip stand will not touch the post with his or her feet, and the shape is suitably adapted to, for example, the slightly inclined backrest of a wheelchair. The post is preferably provided with a handle on a level with the upper body of an average patient, so that the stand provides a good support for the patient when walking. Furthermore, the post is preferably provided with connecting means for connecting the drip stand to a wheelchair, so that the drip stand will move along with a wheelchair without the patient or an attendant having to hold it. The wheels are preferably castors so as to ensure a good mobility of the drip stand in all directions.

The invention also relates to a method for storing mobile drip stands, which drips stands each comprise a frame provided with a post and with front and rear wheels, wherein the front side of the frame of a first drip stand is moved between the rear wheels and under the post of a second drip stand on a flat floor without lifting one of the drip stands.

In another method according to the invention, the frames of the drip stands may on the contrary be lifted, with the frames being positioned one over another, so that the frames are interlocked, for example during transport.

The invention will now be explained in more detail by means of an embodiment as shown in the figures, which is only meant to illustrate the inventive concept, wherein:
Figure 1 is a perspective view of a drip stand according to the invention;
Figure 2 is a perspective view of a group of three drip stands as shown in figure 1 in the stored condition thereof;
Figure 3 is a plan view of the group of figure 3, seen in the direction indicated by the arrow III therein;
Figure 4 is a perspective view of the drip stand of figure 1 in combination with a patient;
Figure 5 is a perspective view of the drip stand of figure 1 in combination with a wheelchair.

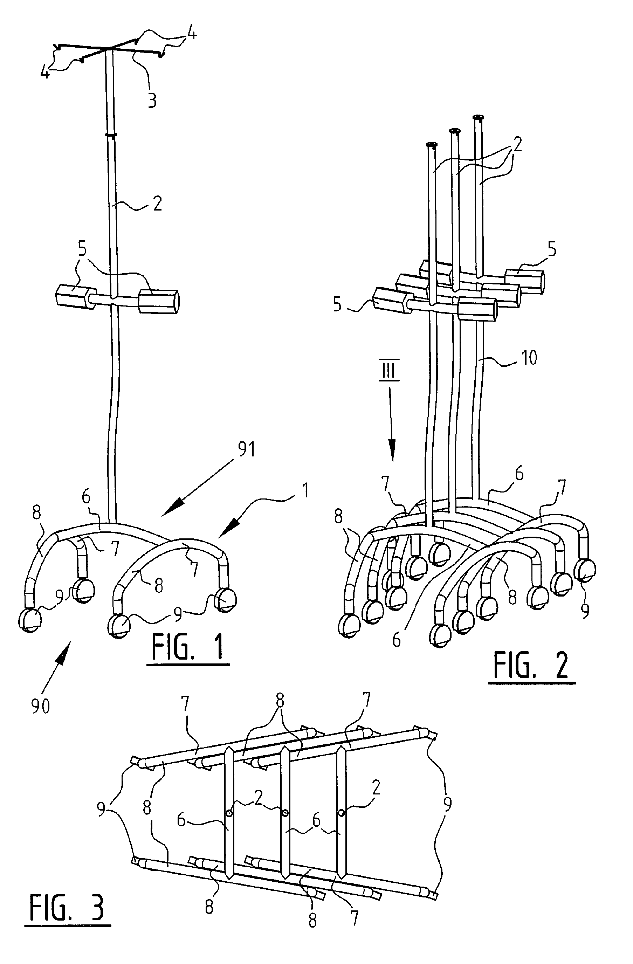

According to figure 1, a drip stand having a front side 90 and a rear side 91 comprises a frame 1, on which a telescopic post 2 is mounted. Mounted to the upper side of the post 2 is a cross 3, which is provided with suspension hooks 4 for drip bags. At about half way its length, the post 2 is furthermore provided with two laterally extending handles 5, by which a user can hold the drip stand and support himself or herself.

The frame 1 comprises a substantially horizontally extending bridge member in 6, in the centre of which the post 2 is mounted. Said bridge member 6 consists of a slightly curved tube member, on which the post 2 is mounted at the highest point thereof. Two other tube members 7 are fixed to the ends of the bridge member 6, substantially transversely thereto, which tube members 7 essentially extend in two opposite directions. In this way a substantially H-shaped frame is obtained. The tube members 7 are slightly curved, and in particular the tube portions 8 that extend towards the front side 90 slope downwards. Furthermore, the tube members 7 as a whole extend in directions towards each other, seen from the rear side 91 in the direction of the front side 90, as is clearly shown in figure 3.

Castors 9 extending in downward direction are mounted to the ends of the tube members 7. The post 2 comprises a portion 10 in the lower half thereof, which is slightly inclined from the frame towards the rear side 91 of the drip stand.

Figure 2 and 3 show three identical drip stands in stored condition. The front side 90 of a drip stand is moved into the opening between the rear wheels 9 with the front wheels 9 thereof, under the bridge member 6 of another drip stand, until the upper sides of the front, downwardly inclined tube portions 8 come into contact with the bottom side of the bridge member 6 of the other drip stand and/or until the outer sides of the slightly converging tube members 7 come into contact with the inner sides of the slightly converging tube members seven of the other drip stand. In this way it is possible to space the posts 2 relatively close together, so that the stored stands will take up little space. In an alternative storage method, the front wheels 9 of a drip stand are lifted over the bridge 6 of the other drip stand and placed between the front tube portions 8 of said other drip stand, so that the drip stands cannot be easily moved apart any longer. This may be desirable when transporting a group of drip stands.

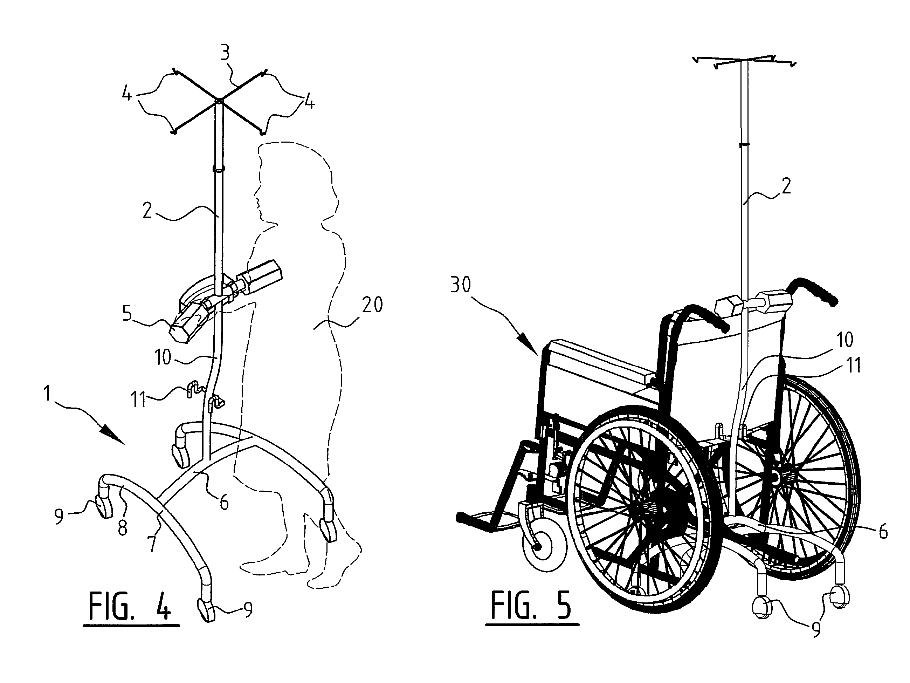

In figure 4 a patient 20 is illustrated in dotted lines, which patient takes hold of the drip stand by the handles 5 and moves around with the drip stand in this way. On account of the comparatively large space that is present between the rear wheels 9 and the inclined portion 10 of the post 2, ample space is provided in which the patient is able to move his or her legs freely.

Figure 5 shows a drip stand that is connected to a wheelchair 30 by means of a hook 11, so that the drip stand will move along with the wheelchair 30 on its own account. The front side 90 of the frame of the drip stand is positioned under the seat of the wheelchair 30 in that situation. The inclined part 10 of the post 2 extends substantially parallel to the backrest of the wheelchair 30, as a result of which the bridge member 6 can be moved maximally under the wheelchair 30. The comparatively large free space between the rear wheels 9 of the stand is now available for the legs of a person who pushes the wheelchair 30, whilst there is little risk of said person's feet coming into contact with the bridge member 6.

## Claims

1. A mobile drip stand comprising a substantially horizontally extending frame (1), which frame (1) comprises a front half with downwardly extending front wheels (9) and a rear half with downwardly extending rear wheels (9), whilst an opening which is wider than the front side (90) of the front half of the frame (1) with the front wheels (9) is present between the rear wheels (9) and a post (2) extends substantially vertically from the frame (1), which post (2) is provided with suspension means (3, 4) for a drip bag, **characterised in that** the post (2) extends from the rear half of the frame (1), and **in that** the height of the upper side of the frame (1) at the front side thereof is lower than the height of the bottom side of the frame (1) under the post (2), such that the front side (90) of the frame (1) of the drip stand can be moved between the rear wheels (9) and under the post (2) of a second, identical drip stand on a flat floor without lifting one of the drip stands.

2. A mobile drip stand according to claim 1, wherein the front side (90) of the frame (1) extends between two spaced-apart front wheels (9).

3. A mobile drip stand according to claim 1 or 2, wherein the frame (1) is made of tube members (6, 7).

4. A mobile drip stand according to claim 3, wherein the frame (1) comprises two more or less parallel, substantially horizontally extending tube members (7), which are interconnected and which are held in spaced-apart relationship by a bridge member (6), in such a manner that the frame (1) is substantially H-shaped, seen from above, with the wheels (9) extending downwards near the ends of the two tube members (7).

5. A mobile drip stand according to claim 4, wherein the post (2) extends from said bridge member (6).

6. A mobile drip stand according to claim 3, 4 or 5, wherein the tube members (7) slope downwards between the post (2) and the front side (90) of the frame (1).

7. A mobile drip stand according to any one of the preceding claims 3-6, wherein the tube members (7) extend in directions towards each other between the post (2) and the front side (90) of the frame (1).

8. A mobile drip stand according to any one of the preceding claims 1-7, wherein the post (2) is slightly upwardly inclined towards the rear side (91) of the frame (1) along at least part of its length.

9. A mobile drip stand according to any one of the preceding claims 1-8, wherein the post (2) is provided with a handle (5) on a level with the upper body of an average person.

10. A mobile drip stand according to any one of the preceding claims 1-9, wherein the post (2) is provided with connecting means (11) for connecting the drip stand to a wheelchair (30).

11. A mobile drip stand according to any one of the preceding claims 1-10, wherein the wheels (9) are castors.

12. A method for storing mobile drip stands, which drip stands comprise a substantially horizontally extending frame (1), which frame (1) comprises a front half with downwardly extending front wheels (9) and a rear half with downwardly extending rear wheels (9), whilst an opening which is wider than the front side (90) of the front half of the frame (1) with the front wheels (9) is present between the rear wheels (9), and which drip stands each comprise a post (2) which extends substantially vertically from the frame (1) and which is provided with suspension means (3, 4) for a drip bag, **characterised in that** the front side (90) of the frame (1) of a first drip stand is moved between the rear wheels (9) and under the post (2) of a second drip stand on a flat floor without lifting one of the drip stands, to which end the height of the upper side of the frame (1) at the front side (90) thereof is lower than the height of the bottom side of the frame (1) under the post (2).
